Europäisches Patentamt

European Patent Office

Office européen des brevets

(19)

(11) **EP 1 616 522 A1**

(12) **EUROPÄISCHE PATENTANMELDUNG**

(43) Veröffentlichungstag:
**18.01.2006 Patentblatt 2006/03**

(51) Int Cl.:
***A61B 5/00*** (2006.01)

(21) Anmeldenummer: **05014063.1**

(22) Anmeldetag: **29.06.2005**

(84) Benannte Vertragsstaaten:
**AT BE BG CH CY CZ DE DK EE ES FI FR GB GR HU IE IS IT LI LT LU MC NL PL PT RO SE SI SK TR**
Benannte Erstreckungsstaaten:
**AL BA HR LV MK YU**

(30) Priorität: **12.07.2004 DE 102004033614**

(71) Anmelder: **Emedics GmbH**
**94032 Passau (DE)**

(72) Erfinder:
• **Assmann, Gunter**
**94032 Passau (DE)**
• **Pöppl, Rudolf**
**94032 Passau (DE)**

(74) Vertreter: **Rothkopf, Ferdinand et al**
**Rothkopf & Theobald**
**Patentanwälte**
**Isartorplatz 5**
**80331 München (DE)**

(54) **Einrichtung und Verfahren zum Abschätzen einer Auftretungswahrscheinlichkeit einer Gesundheitsstörung**

(57) Die Erfindung betrifft eine Einrichtung sowie ein Verfahren zum Abschätzen einer Wahrscheinlichkeit des zukünftigen Auftretens bzw. Nichtauftretens einer Gesundheitsstörung bei einem Individuum. Die genannte Einrichtung weist eine Einlesevorrichtung zum Einlesen jeweils einer zugehörigen Ausprägung mindestens eines charakteristischen Merkmals des Individuums auf. Erfindungsgemäß weist die Einrichtung weiterhin eine Berechnungsvorrichtung zum Berechnen einer Gesamtwahrscheinlichkeit des zukünftigen Auftretens bzw. Nichtauftretens einer von mindestens zwei unterschiedlichen Gesundheitsstörungen aus den eingelesenen Angaben zur jeweiligen Ausprägung des mindestens einen charakteristischen Merkmals auf.

Fig. 1

EP 1 616 522 A1

**Beschreibung**

Die Erfindung betrifft eine Einrichtung und ein Verfahren zum Abschätzen einer Wahrscheinlichkeit eines zukünftigen Auftretens bzw. Nichtauftretens einer Gesundheitsstörung bei einem Individuum. Eine solche Einrichtung weist eine Einlesevorrichtung zum Einlesen jeweils einer zugehörigen Ausprägung mindestens eines charakteristischen Merkmals des Individuums auf. D.h. in dem Fall, in dem lediglich ein charakteristisches Merkmal des Individuums vorliegt, wird eine diesem zugehörige Ausprägung eingelesen; im Fall von mehreren charakteristischen Merkmalen des Individuums, wird bzgl. jedes charakteristischen Merkmals eine jeweils zugehörige Ausprägung eingelesen.

Eine solche Einrichtung und ein solches Verfahren sind im Stand der Technik bekannt. Beispielsweise wird im Internet unter der Adresse "www.chd-taskforce.de" ein sogenannter PROCAM-Risk-Calculator zur Berechnung eines Riskioscores zum Auftreten eines Herzinfarktes bei einer Person angeboten. Zur Ausführung der Risikoberechnung muss der Anwender zugehörige Ausprägungen mehrerer charakteristischer Merkmale der betreffenden Person, nämlich Angaben über dessen Alter, Gewicht, Bluthochdruck, Cholesterinwert, sowie zu Nikotinkonsum und Diabetes Mellitus eingeben. In dem System wurde zuvor eine Wahrscheinlichkeitskorrelation zwischen den möglichen Ausprägungen der o.g. charakteristischen Merkmale und der Wahrscheinlichkeit des zukünftigen Auftretens von Herzinfarkt abgespeichert. Diese Wahrscheinlichkeitskorrelation wurde anhand von Reihenuntersuchungen und Reihenbeobachtungen über einen Zeitraum von 5 bis 20 Jahren von mehreren tausend Probanden und Patienten mit einem bestimmten kardiovaskulären Risikoprofil abgeleitet.

Auf der Grundlage der Risikoeinschätzung für das Auftreten eines Herzinfarktes als Ergebnis der Internetberechnung kann die Testperson geeignete Gegenmaßnahmen, wie etwa eine Verringerung des Nikotinkonsums, des Körpergewichts oder andere unter ärztlicher Konsultation bestimmte präventive Maßnahmen zur Herzinfarktverhinderung ergreifen. Damit sollte sich die Herzinfarktwahrscheinlichkeit der Testperson merklich verringern lassen. Folglich sollte sich aber durch allgemeine Verwendung dieses PROCAM-Risk-Calculators in der Bevölkerung das durchschnittliche Herzinfarktrisko der Bevölkerung verbessern lassen und als Konsequenz sollten die Behandlungskosten des Gesundheitswesens als Ganzes sinken. Allerdings wurden mit dem vorgenannten System in dieser Hinsicht nur begrenzte Erfolge verzeichnet.

Aufgrund dieser Probleme im Stand der Technik liegt daher der Erfindung die Aufgabe zugrunde, eine Einrichtung der eingangs genannten Art bereitzustellen, mit dem eine signifikante Verringerung der Behandlungskosten des Gesundheitswesens erzielt werden kann.

Erfindungsgemäß wird diese Aufgabe mittels der Einrichtung der eingangs genannten Art, im folgenden auch Abschätzeinrichtung bezeichnet, gelöst, welche dadurch gekennzeichnet ist, dass sie eine Berechnungsvorrichtung zum Berechnen einer Gesamtwahrscheinlichkeit des zukünftigen Auftretens bzw. Nichtauftretens einer von mindestens zwei unterschiedlichen Gesundheitsstörungen aus den eingelesenen Angaben zur jeweiligen Ausprägung des mindestens einen charakteristischen Merkmals aufweist.

Diese mindestens zwei vorgegebenen Gesundheitsstörungen können beispielsweise den vorgenannten akuten Herzfarkt und den Lungenkrebs oder den Schlaganfall umfassen. Aus der durch die Einrichtung berechneten Gesamtwahrscheinlichkeit des zukünftigen Auftretens einer oder mehrerer der vorgenannten Gesundheitsstörungen bei dem Individuum lassen sich viel umfassendere Schlussfolgerungen über die Gesundheitserwartung der betreffenden Person als mittels des PROCAM-Risk-Calculators ziehen. Die auf Grundlage einer negativen Prognose ergriffenen präventiven Maßnahmen können damit die Gesundheitserwartung der Person mit einer entsprechend höheren Erfolgsquote verbessern. Durch breite Anwendung der Einrichtung kann damit eine signifikante Verbesserung der durchschnittlichen Gesundheit der Bevölkerung und damit eine signifikante Verringerung der Kosten des Gesundheitswesens erzielt werden.

In einer bevorzugten Ausführungsform ist das Auftreten einer Gesundheitsstörung ein zum Tod des Individuums führendes Auftreten der Gesundheitsstörung und damit ist mittels der Berechnungsvorrichtung die Gesamtwahrscheinlichkeit dafür berechenbar, dass der Tod des Individuums aufgrund einer von mindestens zwei unterschiedlichen Gesundheitsstörungen zukünftig auftritt bzw. nicht auftritt. Dazu wird die Wahrscheinlichkeit des Auftretens einer Gesundheitsstörung oder einer Krankheit mit einer typischen, aus statistischen Erhebungen bekannten Mortalitätsrate für diese Gesundheitsstörung oder Krankheit gewichtet. Das Berechnungsergebnis stellt einen rechnerischen Zusammenhang zwischen Inzidenz und Mortalität einer bestimmten Krankheit her, der in dieser Weise nicht in der medizinisch-epidemiologischen Wissenschaft angewendet wird. Die Mortalität als Gewichtungsfaktor soll die Gefährlichkeit der entsprechenden Krankheit zum Ausdruck bringen. Das Berechnungsergebnis beschreibt somit am ehesten die Wahrscheinlichkeit, dass eine gefährliche Erkrankung beim betreffenden Individuum auftritt bzw. nicht auftritt. Die Bereitstellung dieser Information bewirkt bei den Testpersonen eine besonders hohe Motivation, Präventivmaßnahmen zur Verringerung des Mortalitätsrisikos zu ergreifen. Damit kann mittels dieser Ausführungsform die Volksgesundheit in besonderem Maße verbessert werden.

In einer erfindungsgemäß bevorzugten Ausführungsform weist die Einrichtung weiterhin eine Bereitstellungvorrichtung zum Bereitstellen einer Wahrscheinlichkeitskorrelation zwischen möglichen Ausprägungen des mindestens einen charakteristischen Merkmals und der Wahrscheinlichkeit des zukünftigen Auftretens bzw. Nichtauftretens einer von mindestens zwei vorgegebenen Gesundheitsstörungen auf und die Gesamtwahrscheinlichkeit ist mittels der Berechnungsvorrichtung aus der Wahrscheinlichkeitskorrelation berechenbar. Die zugehörige Wahrscheinlichkeitskorrelation wird aus entsprechenden statistischen Erhebungen und Studien bzgl. der Auftretenshäufigkeit dieser Gesundheitsstörungen anhand einer ausreichend großen Anzahl von Probanden gewonnen. Die Wahrscheinlichkeitskorrelation kann entweder in Form einer Zuordnungstabelle zwischen den möglichen Ausprägungen des oder der charakteristischen Merkmals bzw. Merkmale und entsprechenden Wahrscheinlichkeitswerten oder in Form einer die entsprechende Zuordnung definierenden mathematischen Funktion vorgesehen werden.

Als erfindungsgemäß vorteilhaft hat sich erwiesen, wenn mittels der Bereitstellungsvorrichtung die Wahrscheinlichkeitskorrelation durch Kombination einer ersten in einer Speichervorrichtung abgespeicherten Einzelwahrscheinlichkeitskorrelation zwischen möglichen Ausprägungen mindestens eines des mindestens einen charakteristischen Merkmals und der Wahrscheinlichkeit des zukünftigen Auftretens bzw. Nichtauftretens mindestens einer ersten vorgegebenen Gesundheitsstörung mit mindestens einer zweiten in der Speichervorrichung abgespeicherten Einzewahrscheinlichkeitskorrelation zwischen möglichen Ausprägungen mindestens eines des mindestens einen charakteristischen Merkmals und der Wahrscheinlichkeit des zukünftigen Auftretens bzw. des Nichtauftretens einer zweiten vorgegebenen Gesundheitsstörung in der Abschätzeinrichtung bereitstellbar ist. Dadurch, dass die Auftretenswahrscheinlichkeit bzw. Nichtauftretenswahrscheinlichkeit jeder der erfaßten Gesundheitsstörungen über eine Einzelwahrscheinlichkeitskorrelation in der Abschätzeinrichtung abgespeichert wird, ist es möglich, neue Forschungsergebnisse bzgl. einzelner Gesundheitsstörungen problemlos aufzunehmen. Das heißt, sollten neue Studien bzgl. einer bestimmten Gesundheitsstörung eine veränderte Wahrscheinlichkeitskorrelation für diese Gesundheitsstörung zum Ergebnis haben, so muss nur die entsprechende Einzelwahrscheinlichkeitskorrelation in der Abschätzeinrichtung überschrieben werden. Die restlichen Einzelwahrscheinlichkeitskorrelationen werden nicht verändert und die Abschätzeinrichtung erzeugt selbstständig die neue resultierende Wahrscheinlichkeitskorrelation zur Berechnung der Gesamtwahrscheinlichkeit.

Weiterhin hat es sich als vorteilhaft erwiesen, wenn die Bereitstellungsvorrichtung eine Speichervorrichtung zum Abspeichern einer über eine Eingabevorrichtung eingelesenen Wahrscheinlichkeitskorrelation aufweist bzw. ist. In diesem Fall muss die Abschätzeinrichtung nicht mehr erst die Wahrscheinlichkeitskorrelation aus den entsprechenden Einzelwahrscheinlichkeitskorrelationen erzeugen, bevor sie die Gesamtwahrscheinlichkeit berechnen kann. Damit läßt sich mit dieser Ausführungsform das Rechenverfahren entsprechend vereinfachen, was zu einem besonders schnellen Bereitstellen der Berechnungsergebnisse für den Anwender führt.

In einer zweckmäßigen Ausführungsform stellt die mittels der Berechnungsvorrichtung berechenbare Gesamtwahrscheinlichkeit die Wahrscheinlichkeit des zukünftigen Auftretens bzw. Nichtauftretens einer Gesundheitsstörung innerhalb eines vorgegebenen Zeitraums dar. Vorzugsweise kann der Benutzer diesen Zeitraum selbst vorgeben. Geeignete Prognosezeiträume können etwa 5, 10, 20 oder 30 Jahre sein. Ein Gesundheitsindex wird definiert als das Gesundbleiben zu x % in 5 / 10 / 20 ... Jahren. Damit wird die Brauchbarkeit des erfindungsgemäßen Verfahren weiter erhöht. Bei einer jungen Testperson ist es nämlich oft äussagekräftiger, einen längeren Prognosezeitraum zu wählen, als bei einer älteren Testperson, da bei einem jungen Menschen selbst ein ungesunder Lebensstil die Krankheitswahrscheinlichkeit innerhalb eines kurzen Zeintraums von z.B. 5 Jahren möglicherweise nicht wesentlich erhöht.

Weiterhin ist es erfindungsgemäß zweckmäßig, wenn die Einlesevorrichtung eine Dateneingabevorrichtung zur Eingabe der zugehörigen Ausprägung eines charakteristischen Merkmals des Individuums aufweist. Eine solche Dateneingabevorrichtung kann eine Computertastatur, eine Computermaus, aber auch eine bei Personal Digital Assistant's (PDA's) übliche Eingabeeinrichtung sein. Die mittels externen Geräten, wie etwa einem Glucose/Cholesterin-Meßgerät, einem Blutdruckmessgerät oder einem Urin-Teststreifen gemessenen Werte der charakteristischen Merkmale lassen sich damit ohne großen Aufwand in die Abschätzeinrichtung eingeben. Die zur Bestimmung der Meßwerte benötigten Meßgeräte werden dem Benutzer vorzugsweise zusammen mit der Abschätzeinrichtung zur Verfügung gestellt. Beispielsweise können Abschätzeinrichtung und Messgeräte zusammen in einem eigens darauf abgestimmten Köfferchen angeboten werden.

In einer bevorzugten Ausführungsform weist die Einlesevorrichtung eine Meßvorrichtung zum direkten Bestimmen der zugehörigen Ausprägung eines charakteristischen Merkmals des Individuums auf. Diese Meßvorrichtung kann ein automatisches Glucose-/Cholesterin-Meßgerät und/oder ein Blutdruckmessgerät etc. umfassen. Diese Messvorrichtung ist vorzugsweise integral mit der Abschätzeinrichtung ausgebildet, so dass keine zusätzlichen Meßgeräte zur Bestimmung der wichtigsten charakteristischen Merkmalswerte benötigt werden und die Meßwerte direkt in die Abschätzeinrichtung eingelesen werden können. Damit ist die Abschätzeinrichtung besonders benutzerfreundlich.

Weiterhin ist es erfindungsgemäß vorteilhaft, wenn mittels der Einlesevorrichtung die zugehörige Ausprägung eines charakteristischen Merkmals des Individuums als Zahlenwert einlesbar ist. Diesbezüglich sind insbesondere charakteristische Merkmale wie Alter, Gewicht, Blutdruckwerte und/oder Glucose-/Cholesterinwerte geeignet. Erfindungsgemäß kann mittels der Einlesevorrichung die zugehörige Ausprägung eines charakteristischen Merkmals des Individuums aber auch eine qualitative Angabe ein lesbar sein. Solche Angaben können etwa Geschlecht und Hautfarbe der Person betreffen, aber auch Angaben in Textform über ein Ausmaß einer Ausprägung krankheitsbezogener Risikofaktoren, sowie weiter Informationen aus Arztbriefen, Impfstatusberichten etc. umfassen. Damit läßt sich eine große Bandbreite an charakteristischen Merkmalen des Individuums berücksichtigen, wodurch die Vorhersagegenauigkeit des Systems weiter optimiert werden kann.

In einer bevorzugten Ausführungsform der Erfindung ist mittels der Berechnungsvorrichtung die Gesamtwahrscheinlichkeit des zukünftigen Auftretens bzw. Nichtauftretens einer von nur die Gesundheit eines bestimmten Organs betreffenden Gesundheitsstörungen berechenbar und damit gibt die berechnete Gesamtwahrscheinlichkeit die Wahrscheinlichkeit für das Individuum an, an dem betreffenden Organ zu erkranken bzw. nicht zu erkranken. Beispielsweise kann die Wahrscheinlichkeitskorrelation nur die Gesundheit des Herzkreislaufsystems, des Atmungssystems, des gastrointestinalen Systems, des Urogenitalsystems oder der Haut betreffen. Auch kann die Wahrscheinlichkeitskorrelation nur rheumatische oder neurologische Krankheiten umfassen. In dem Fall, in dem die Wahrscheinlichkeitskorrelation z.B. nur die Gesundheit des Herzkreislaufsystems betrifft, können folgende Gesundheitsstörungen erfaßt werden: akuter Herzinfarkt, koronare Herzkrankheit, Vorhofflimmern, Herzinsuffizienz, tiefe Beinvenenthrombose, Lungenembolie, Varizen der unteren Extremität, arterielle Verschlußkrankheit der Beine (claudicatio intermittens), Apoplexie des Gehirns und/oder Aortenstenose. Durch die Aufschlüsselung der Erkrankungswahrscheinlichkeit nach einzelnen Organen im Rahmen der Ausgabe eines sogenannten Organindexes können eigene auf die bestimmten Organe zugeschnittene Präventionspläne aufgestellt werden. Damit wird eine zielgenaue Präventionsstrategie ermöglicht, wodurch der Gesamterfolg in Bezug auf eine Verbesserung der Gesundheit der Bevölkerung weiter optimiert werden kann.

Weiterhin ist es erfindungsgemäß vorteilhaft, wenn die mindestens zwei Gesundheitsstörungen mindestens zwei unterschiedliche Organe/Organsysteme betreffen und damit die berechnete Gesamtwahrscheinlichkeit die Wahrscheinlichkeit für das Individuum angibt, an einem der mindestens zwei unterschiedlichen Organe/Organsysteme zu erkranken bzw. nicht zu erkranken. Jede der mindestens zwei Gesundheitsstörungen kann genau ein Organ bzw. Organsystem bzw. auch mehr als ein Organ bzw. Organsystem betreffen. Insbesondere können die Gesundheitsstörungen auch 3, 4 oder mehr Organe betreffen.

Es ist weiterhin erfindungsgemäß zweckmäßig, wenn mittels der Berechnungsvorrichtung die Gesamtwahrscheinlichkeit des zukünftigen Auftretens bzw. Nichtauftreten einer von bestimmten für die Abschätzung der Gesamtgesundheit des Individuums wesentlichen Gesundheitsstörungen berechenbar ist und damit die berechnete Gesamtwahrscheinlichkeit die Wahrscheinlichkeit für das Individuum angibt, insgesamt gesund bzw. nicht gesund zu bleiben. Die Wahrscheinlichkeit, insgesamt gesund zu bleiben, wird als Gesundheitsindex bezeichnet. Damit wird der Testperson ein alle Aspekte seiner zukünftigen Gesundheitsentwicklung subsumierender Ergebniswert an die Hand gegeben. Dieser Wert ermöglicht der Testperson bei einem schnellen Gesundheitscheck eine sofortige Einschätzung seiner Gesundheitserwartung, d.h. sozusagen "auf den ersten Blick". Eine langwierige Auswertung von Einzelergebnissen ist zumindest zunächst nicht nötig. Eine solche Auswertung wird erst dann nötig, wenn das Ergebnis so schlecht ist, dass Präventivmaßnahmen ergriffen werden müssen, und wird dann oftmals von einem Arzt vorgenommen. Durch diese Gesamtgesundheitsabschätzung mittels eines Ergebniswertes erhöht sich somit die Bedienungsfreundlichkeit der Abschätzeinrichtung und damit die Akzeptanz in der Bevölkerung. Der Gesundheitscheck kann somit von den Testpersonen in regelmäßigen Abständen ohne Anleitung durch einen Arzt vorgenommen werden.

In einer bevorzugten Ausführungsform umfassen die für die Abschätzung der Gesamtgesundheit des Individuums wesentlichen Gesundheitsstörungen den akuten Herzinfarkt und die Herzschwäche, den Lungen- und Dickdarmkrebs, den Schlaganfall und/oder Diabetes Mellitus, sowie im Falle einer weiblichen Person insbesondere den Brustkrebs der Frau. Diese Gesundheitsstörungen weisen eine sehr hohe Mortalitätsrate auf und sind damit zur Abschätzung der Gesamtgesundheit besonders repräsentativ. Durch die Auswahl dieser begrenzten Anzahl von zur Abschätzung der Gesamtgesundheit herangezogenen Gesundheitsstörungen läßt sich ein optimales Verhältnis zwischen Aufwand und Nutzen erzielen. Durch die begrenzte Anzahl von berücksichtigten Gesundheitsstörungen muss nur eine geringe Anzahl von charakteristischen Merkmalen bestimmt werden. Der Meß- und Eingabeaufwand wird damit auf ein Minimum reduziert. Gleichzeitig erlauben die berücksichtigten Gesundheitsstörungen aber immer noch eine aussagekräftige Abschätzung der Gesundheitserwartung.

Es ist darüber hinaus erfindungsgemäß zweckmäßig, wenn die Einrichtung weiterhin eine Datenübertragungsvorrichtung zum Empfangen und / oder Versenden von Daten von einem bzw. an einen externen Datenspeicher aufweist. So können etwa in einer Arztpraxis erhobene aktuelle Messwerte von charakteristischen Merkmalen über z.B. eine Infrarotschnittstelle, das Mobilfunknetz oder das Internet an die Abschätzvorrichtung übertragen werden. Ein mühsames Eingeben der Werte erübrigt sich damit. Auch können von der Abschätzeinrichtung berechnete Wahrscheinlichkeitsergebnisse über die Gesundheitserwartung der Testperson automatisch an z.B. das Computersystem eines behandelnden Arztes oder die Krankenversicherung, die bei positiven Ergebnissen dem Versicherten möglicherweise einen Rabatt einräumt, übertragen werden. Durch diese Maßnahme lässt sich die Abschätzeinrichtung besonders effizient betreiben.

Die Erfindung betrifft ferner ein Verfahren zum Abschätzen einer Wahrscheinlichkeit eines zukünftigen Auftretens bzw. Nichtauftretens einer Gesundheitsstörung bei einem Individuum, bei dem jeweils eine zugehörige Ausprägung mindestens eines charakteristischen Merkmals des Individuums in eine Abschätzeinrichtung eingelesen wird. Wie bereits vorstehend erwähnt, werden mit vorbekannten Verfahren dieser Art nur begrenzte Erfolge hinsichtlich der Möglichkeit, die Behandlungskosten des Gesundheitswesens zu verringern, erzielt.

Die Erfindung stellt sich mithin die Aufgabe, eine Einrichtung der vorgenannten Art bereitzustellen, mit der eine signifikante Verringerung der Behandlungskosten des Gesundheitswesens erzielt werden kann.

Diese Aufgabe wird nach der Erfindung mittels des Verfahrens der vorgenannten Art gelöst, bei dem aus dem mindestens einen charakteristischen Merkmal des Individuums eine Gesamtwahrscheinlichkeit des zukünftigen Auftretens bzw. Nichtauftretens einer von mindestens zwei unterschiedlichen Gesundheitsstörungen berechnet wird.

Wie bereits oben unter Bezugnahme auf die erfindungsgemäße Einrichtung erörtert, lassen sich mittels der erfindungsgemäßen Einrichtung viel umfassendere Schlussfolgerungen über die Gesundheitserwartung der betreffenden Person ziehen, weshalb eine breite Verwendung des Verfahrens eine signifikante Verbesserung der durchschnittlichen Gesundheit der Bevölkerung erwarten läßt, wodurch wiederum die Behandlungskosten des Gesundheitswesens als Ganzes gesenkt werden können.

Als erfindungsgemäß zweckmäßig hat sich erwiesen, wenn das Auftreten einer Gesundheitsstörung ein zum Tod des Individuums führendes Auftreten der Gesundheitsstörung ist und damit die Abschätzeinrichtung die Gesamtwahrscheinlichkeit dafür berechnet, dass der Tod des Individuums aufgrund einer von mindestens zwei unterschiedlichen Gesundheitsstörungen zukünftig auftritt bzw. nicht auftritt. Zur näheren Erörterung dieser Ausführungsform wird auf die vorstehenden Ausführungen bzgl. der entsprechenden erfindungsgemäßen Einrichtung verwiesen.

Weiterhin ist es erfindungsgemäß vorteilhaft, wenn zur Berechnung der Gesamtwahrscheinlichkeit eine Wahrscheinlichkeitskorrelation zwischen möglichen Ausprägungen des mindestens einen charakteristischen Merkmals und der Wahrscheinlichkeit des zukünftigen Auftretens bzw. des Nichtauftretes einer von mindestens zwei vorgegebenen Gesundheitsstörungen in der Abschätzeinrichtung bereitgestellt wird. Zur näheren Erörterung dieser Ausführungsform wird auf die vorstehenden Ausführungen bzgl. der entsprechenden erfindungsgemäßen Einrichtung verwiesen.

Als erfindungsgemäß vorteilhaft hat sich erwiesen, wenn die Wahrscheinlichkeitskorrelation durch Kombination einer ersten abgespeicherten Einzelwahrscheinlichkeitskorrelation zwischen möglichen Ausprägungen mindestens eines des mindestens einen charakteristischen Merkmals und der Wahrscheinlichkeit des zukünftigen Auftretens bzw. Nichtauftretens mindestens einer ersten vorgegebenen Gesundheitsstörung mit mindestens einer zweiten abgespeicherten Einzelwahrscheinlichkeitskorrelation zwischen möglichen Ausprägungen mindestens eines des mindestens einen charakteristischen Merkmals und der Wahrscheinlichkeit des zukünftigen Auftretens bzw. des Nichtauftretens einer zweiten vorgegebenen Gesundheitsstörung in der Abschätzeinrichtung bereitgestellt wird. Zur näheren Erörterung dieser Ausführungsform wird auf die vorstehenden Ausführungen bzgl. der entsprechenden erfindungsgemäßen Einrichtung verwiesen.

Weiterhin hat es sich als vorteilhaft erwiesen, wenn die Wahrscheinlichkeitskorrelation von einer Speicherungsvorrichtung in der Abschätzeinrichtung bereitgestellt wird. Auch hier wird zur näheren Erörterung auf die vorstehenden Ausführungen bzgl. der entsprechenden erfindungsgemäßen Einrichtung verwiesen.

In einer zweckmäßigen Ausführungsform gibt die von der Abschätzeinrichtung berechnete Gesamtwahrscheinlichkeit die Wahrscheinlichkeit des zukünftigen Auftretens bzw. Nichtauftretens einer Gesundheitsstörung innerhalb eines vorgegebenen Zeitraums an. Zur näheren Erörterung dieser Ausführungsform wird auch hier auf die vorstehenden Ausführungen bzgl. der entsprechenden erfindungsgemäßen Einrichtung verwiesen.

Weiterhin ist es erfindungsgemäß zweckmäßig, wenn die zugehörige Ausprägung eines charakteristischen Merkmals des Individuums über eine Dateneingabevorrichtung in die Abschätzeinrichtung eingelesen wird. Auch hier wird auf die vorstehenden Ausführungen bzgl. der entsprechenden erfindungsgemäßen Einrichtung zur näheren Erörterung verwiesen.

In einer bevorzugten Ausführungsform wird die zugehörige Ausprägung eines charakteristischen Merkmals des Individuums über eine Messvorrichtung zum direkten Bestimmen der Ausprägung in die Abschätzeinrichtung eingelesen. Zur näheren Erörterung dieser Ausführungsform wird auf die vorstehenden Ausführungen bzgl. der entsprechenden erfindungsgemäßen Einrichtung verwiesen.

Weiterhin ist es erfindungsgemäß vorteilhaft, wenn die zugehörige Ausprägung eines charakteristischen Merkmals des Individuums ein Zahlenwert ist. Erfindungsgemäß kann die zugehörige Ausprägung eines charakteristischen Merkmals des Individuums aber auch eine qualitative Angabe sein. Zur näheren Erörterung dieser Ausführungsformen wird auf die vorstehenden Ausführungen bzgl. der entsprechenden erfindungsgemäßen Einrichtung verwiesen.

In einer bevorzugten Ausführungsform der Erfindung wird die Berechnung der Gesamtwahrscheinlichkeit nur bezüglich von die Gesundheit eines bestimmten Organs betreffenden Gesundheitsstörungen aufgeführt und damit gibt die berechnete Gesamtwahrscheinlichkeit die Wahrscheinlichkeit für das Individuum an, an dem betreffenden Organ zu erkranken bzw. nicht zu erkranken. Zur näheren Erörterung dieser Ausführungsform wird auch hier auf die vorstehenden Ausführungen bzgl. der entsprechenden erfindungsgemäßen Einrichtung verwiesen.

Weiterhin ist es erfindungsgemäß vorteilhaft, wenn die mindestens zwei Gesundheitsstörungen mindestens zwei unterschiedliche Organe/Organsysteme betreffen und damit die berechnete Gesamtwahrscheinlichkeit die Wahrscheinlichkeit für das Individuum angibt, an einem der mindestens zwei unterschiedlichen Organe/Organsysteme zu erkranken bzw. nicht zu erkranken. Zur näheren Erörterung dieser Ausführungsform wird auch hier auf die vorstehenden Ausführungen bzgl. der entsprechenden erfindungsgemäßen Einrichtung verwiesen.

Weiterhin ist es erfindungsgemäß zweckmäßig, wenn die Berechnung der Gesamtwahrscheinlichkeit nur bezüglich bestimmter, für die Abschätzung der Gesamtgesundheit des Individuums wesentlicher Gesundheitsstörungen ausgeführt wird und damit die berechnete Gesamtwahrscheinlichkeit die Wahrscheinlichkeit für das Individuum angibt, insgesamt gesund bzw. nicht gesund zu bleiben. Auch hier wird zur näheren Erörterung auf die vorstehenden Ausführungen bzgl. der entsprechenden erfindungsgemäßen Einrichtung verwiesen.

In einer bevorzugten Ausführungsform umfassen die für die Abschätzung der Gesamtgesundheit des Individuums wesentlichen Gesundheitsstörungen den akuten Herzinfarkt und die Herzschwäche, den Lungen- und Dickdarmkrebs, den Schlaganfall und/oder Diabetes Mellitus, sowie im Falle einer weiblichen Person insbesondere den Brustkrebs der Frau. Zur näheren Erörterung dieser Ausführungsform wird auf die vorstehenden Ausführungen bzgl. der entsprechenden erfindungsgemäßen Einrichtung verwiesen.

Es ist darüber hinaus erfindungsgemäß zweckmäßig, wenn weiterhin Daten über eine Datenübertragungsvorrichtung von einem bzw. an einen externen Datenspeicher empfangen und/oder versendet werden. Zur näheren Erörterung dieser Ausführungsform wird auf die vorstehenden Ausführungen bzgl. der entsprechenden erfindungsgemäßen Einrichtung verwiesen.

Die Erfindung betrifft femer ein Computer-Programm mit Programmcode zur Durchführung des erfindungsgemäßen Verfahrens sowie ein Computer-Programm-Produkt mit auf einem maschinenlesbaren Träger, insbesondere einer CD, gespeichertem Programmcode zur Durchführung des erfindungsgemäßen Verfahrens.

Nachstehend wird die Erfindung unter Bezugnahme auf die Zeichnung, auf die hinsichtlich aller erfindungswesentlichen Einzelheiten ausdrücklich verwiesen wird, erläutert. In der Zeichnung zeigt:

Fig. 1 ein eine Ausführungsform der erfindungsgemäßen Einrichtung zum Abschätzen einer Wahrscheinlichkeit des zukünftigen Auftretens bzw. Nichtauftretens einer Gesundheitsstörung bei einem Individuum darstellendes Blockdiagramm,

Fig. 2 ein eine Ausführungsform des erfindungsgemäßen Verfahrens zum Abschätzen einer Wahrscheinlichkeit des zukünftigen Auftretens bzw. Nichtauftretens einer Gesundheitsstörung bei einem Individuum veranschaulichendes Flussdiagramm, sowie

Fig.3 eine schematische Darstellung einer weiteren Ausführungsform der erfindungsgemäßen Abschätzeinrichtung.

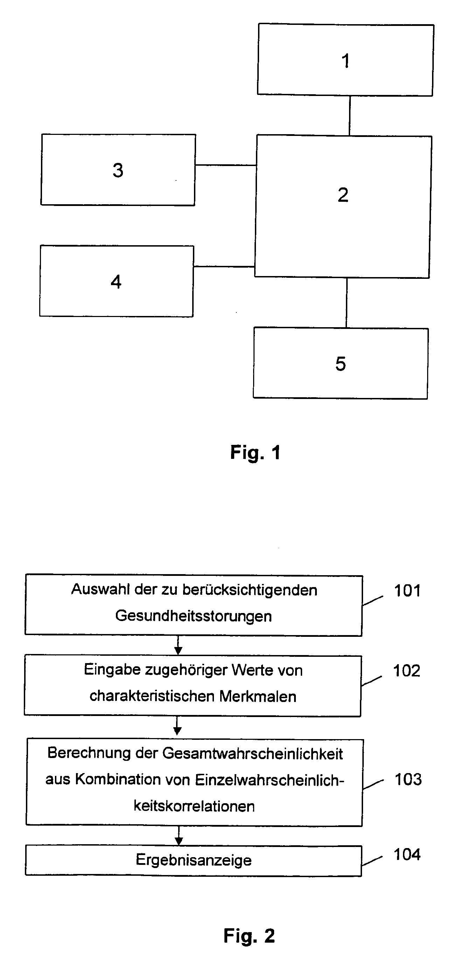

Nachstehend wird die Funktionsweise einer Ausführungsform der erfindungsgemäßen Abschätzvorrichtung unter Bezugnahme auf Fig. 1 und Fig. 2 beschrieben. Zunächst gibt die Abschätzvorrichtung dem Benutzer die Möglichkeit die für eine vorzunehmende Gesundheitsabschätzung zugrundezulegenden Gesundheitsstörungen über eine Dateneingabeeinrichtung 1 festzulegen (Schritt 101). Dabei kann der Benutzer aus den in den nachstehenden Tab. 1 und Tab. 2 nach Organfunktionen geordnet aufgeführten Gesundheitsstörungen wählen.

Tab. 1

| Lfd. Nr. | Gesundheitsstörung |
|---|---|
| Herzkreislaufsystem | |
| 1 | Akuter Herzinfarkt |
| 2 | Koronare Herzkrankheit |
| 3 | Vorhofflimmern |
| 4 | Herzinsuffizienz |
| 5 | Tiefe Beinvenenthrombose |
| 6 | Lungenembolie |
| 7 | Varizen der unteren Extemität |
| 8 | Arterielle Verschlusskrankheit der Beine (Claudicatio intermittens) |
| 9 | Apoplexie des Gehirns |
| 10 | Aortenstenose |

Tabelle fortgesetzt

| Atmungssystem | |
|---|---|
| 11 | Asthma bronchiale |
| 12 | Chronisch obstruktive Lungenerkrankung |
| 13 | Bronchialcarcinom |
| 14 | Lungenemphysem |
| Gastrointestinales System | |
| 15 | Refluxkrankheit der Speiseröhre |
| 16 | Gastroduodenale Ulcuskrankheit |
| 17 | Ösophaguscarcinom |
| 18 | Magencarcinom |
| 19 | Entzündliche Darmerkrankung (M. Crohn / Colitis ulcerosa) |
| 20 | Divertikel des Dickdarms |
| 21 | Koloncarcinom |
| 22 | Pankreatitis |
| 23 | Pankreascarcinom |
| 24 | Äthyltoxische Hepatitis und Leberzirrhose |
| 25 | Cholezystolithiasis |
| 26 | Hämorrhidalleiden |

Tab. 2

| Lfd. Nr. | Gesundheitsstörung |
|---|---|
| Urogenitalsystem | |
| 27 | Nephrolithiasis |
| 28 | Chronische Niereninsuffizienz bei Diabetes mellitus |
| 29 | Nierenzellcarcinom |
| 30 | Prostatacarcinom |
| 31 | Mammacarcinom |
| 32 | Uteruscarcinom |
| 33 | Uterusmyom |
| Hautkrankheiten | |
| 34 | Atopisches Ekzem |
| 35 | Hautkrebs (incl. Malignes Melanom) |
| Rheumatische Erkrankungen | |
| 36 | Chronische Polyarthritis |
| 37 | Degeneratives BWS-LWS-Syndrom |
| 38 | Osteoporose |
| 39 | Coxarhthrose |
| Neurologie | |
| 40 | M. Alzheimer |

**EP 1 616 522 A1**

Tabelle fortgesetzt

| Neurologie | |
|---|---|
| 41 | Epilepsie |
| Endokrine Erkrankungen | |
| 42 | Diabetes mellitus |
| 43 | Struma der Schilddrüse |

Weiterhin bietet das System die Option, eine sogenannte aufwandsoptimierte Gesundheitsabschätzung anzuwählen. Dabei werden der Abschätzung nur folgende Gesundheitsstörungen mit einer hohen Mortalitätsrate zugrundegelegt: akuter Herzinfarkt, Herzschwäche, Lungen- und Dickdarmkrebs, Schlaganfall, Diabetes Mellitus, sowie zusätzlich bei einer weiblichen Testperson der Brustkrebs der Frau. Darüber hinaus besteht natürlich die Möglichkeit, alle 43 in den Tab. 1 und 2 aufgeführten Gesundheitsstörungen für die nachfolgende Abschätzung anzuwählen.

Nachdem der Anwender n Gesundheitsstörungen ausgewählt hat, wird er aufgefordert, über die Dateneingabevorrichtung 1 zugehörige Ausprägungen oder Werte von zur Abschätzung seiner Gesundheitserwartung auf Grundlage der zuvor ausgewählten Gesundheitsstörungen benötigten charakteristischen Merkmalen einzugeben (Schritt 102). Solche charakteristische Merkmale können Alter, Gewicht, Geschlecht, Bluthochdruck, Nikotinkonsum und Cholesterinspiegel umfassen. Bei einer optionalen Ausführungsform der erfindungsgemäßen Abschätzeinrichtung kann der Anwender als nächsten Schritt eine den Prognosezeitraum Z der auszuführenden Gesundheitsabschätzung definierende Anzahl von Jahren eingeben. Damit wird der Zeitraum in Jahren definiert, innerhalb dem das Auftreten einer Gesundheitsstörung mit einer gewissen Wahrscheinlichkeit als Ergebnis der Abschätzung erwartet wird. Bei der bevorzugten Ausführungsform der Erfindung wählt der Anwender den Prognosezeitraum Z nicht selbst aus. Dieser wird vielmehr von der Abschätzeinrichtung vorgegeben.

Daraufhin wird von einem Mikroprozessor 2 anhand der folgenden Formel (I) die Gesamtwahrscheinlichkeit $P_{Ges}$ für den Fall berechnet, dass eine der ausgewählten Gesundheitsstörungen bei der Testperson innerhalb des betreffenden Prognosezeitraums Z nicht auftritt.

$$P_{Ges} = 1 - Z \times \sum_{i=1}^{n} I_i(x_{i1}, x_{i2}, x_{i3}, ...) \qquad (I)$$

wobei $x_{i1}$, $x_{i2}$, $x_{i3}$, ... die eingegebenen Werte der der jeweiligen Gesundheitsstörung i zugeordneten charakteristischen Merkmale und $I_i$ die in einer Speichervorrichtung 3 abgespeicherten individuellen Inzidenzfunktionen oder Einzelwahrscheinlichkeitskorrelationen sind, die jeweils den Zusammenhang zwischen den möglichen Werten der charakteristischen Merkmale und der Wahrscheinlichkeit des Auftretens, d.h. der Inzidenz der Gesundheitsstörung i innerhalb eines Jahres definieren. Die individuellen Inzidenzfunktionen $I_i$ werden anhand von statistischen Erhebungen und wissenschaftlichen Studien bestimmt und sind in der Speichervorrichtung 3 fest abgespeichert. Sie können aber bei Bedarf abgeändert werden, etwa um neue Forschungsergebnisse zu berücksichtigen. Weiterhin bezeichnet n die Anzahl der ausgewählten Gesundheitsstörungen i und Z den maßgeblichen Prognosezeitraum in Jahren.

Weiterhin wird anhand der nun folgenden Formel (II) von dem Mikroprozessor 2 auch der sogenannte Gesundheitsindex berechnet, das heißt die Gesamtwahrscheinlichkeit dafür, dass die Testperson innerhalb des angegebenen Prognosezeitraums Z nicht stirbt.

$$G = 1 - Z \times \left( \sum_{i=1}^{n} \left[ I_i(x_{i1}, x_{i2}, x_{i3}, ...) \times \frac{M_{Bi}}{I_{Bi}} \right] - \sum_{i=1}^{n} M_{Bi} + M_{Bges} \right) \qquad (II)$$

wobei $x_{i1}$, $x_{i2}$, $x_{i3}$, ... wiederum die eingegebenen Werte der der jeweiligen Gesundheitsstörung i zugeordneten charakteristischen Merkmale und $I_i$ die in der Speichervorrichtung 3 abgespeicherten individuellen Inzidenzfunktionen, die jeweils den Zusammenhang zwischen möglichen Werten der charakteristischen Merkmale und der Wahrscheinlichkeit des Auftretens, d.h. der Inzidenz der Gesundheitsstörung i innerhalb eines Jahres definieren. $M_{Bi}$ ist die jeweilige durch-

8

schnittliche Bevölkerungsmortalität bezüglich der Gesunheitsstörung i, d.h. die durchschnittliche Wahrscheinlichkeit für ein beliebiges Mitglied der Bevölkerung, innerhalb eines Jahres an der Gesundheitsstörung i zu sterben. $I_{Bi}$ bezeichnet die Bevölkerungsinzidenz bezüglich der Gesundheitsstörung i, d.h. die Wahrscheinlichkeit für ein beliebiges Mitglied der Bevölkerung, innerhalb eines Jahres an der Gesundheitsstörung i zu erkranken. Der Ausdruck [ $I_i(x_{i1}, x_{i2}, x_{i3}, ...) \times M_{Bi}$ / $I_{Bi}$ ] innerhalb der Formel (II) stellt somit eine Einzelwahrscheinlichkeitskorrelation zwischen den eingegebenen Werten der Gesundheitsstörung i und der Wahrscheinlichkeit des zukünftigen Auftretens der Gesundheitsstörung i mit tödlichem Ausgang innerhalb eines Jahres dar. $M_{Bges}$ bezeichnet die Gesamtmortalität der Bevölkerung innerhalb eines Jahres, d.h. die Wahrscheinlichkeit dafür, dass ein beliebiges Mitglied der Bevölkerung innerhalb eines Jahres an einer beliebigen Gesundheitsstörung stirbt. Der Ausdruck [ $\Sigma M_{Bi} + M_B g_{es}$ ] innerhalb der Formel (II) berücksichtigt den Anteil der in der Formel nicht individuell berücksichtigten Gesundheitsstörungen am individuellen Gesamtmortalitätsrisiko. Die Bevölkerungsmortalitäten $M_{Bi}$, die Bevölkerungsinzidenzen $I_{Bi}$ sowie die Gesamtmortalität $M_{Bges}$ der Bevökerung werden anhand statistischer Erhebungen bestimmt und sind ebenfalls in der Speichervorrichtung 3 fest abgespeichert. Weiterhin bezeichnet n die Anzahl der ausgewählten Gesundheitsstörungen i und Z den maßgeblichen Prognosezeitraum in Jahren.

Weiterhin werden analog zur Formel (II) auch sogenannte Organindizes für alle in Tab. 2 aufgeführten Organsysteme berechnet, indem in der Formel anstatt aller n ausgewählter Gesundheitsstörungen i jeweils lediglich die Gesundheitsstörungen i berücksichtigt werden, die gemäß Tab. 2 einem bestimmten Organ zugeordnet sind; anstatt $M_{Bges}$ wird in diesem Fall die Gesamtmortalität der Bevölkerung bzgl. des betreffenden Organs innerhalb eines Jahres in die Formel (II) eingesetzt. Die daraus erhaltenen Organindexwerte geben damit die Wahrscheinlichkeit dafür an, dass die Testperson innerhalb des Prognosezeitraums Z nicht an einer das jeweilige Organ betreffenden Gesundheitsstörung stirbt. Weiterhin wird auch ein sogenannter Organfunktionsindex berechnet, der die Wahrscheinlichkeit des Auftretens einer bestimmten, ein Organ betreffenden Gesundheitsstörung bezeichnet.

Die berechneten Werte für den Gesundheitsindex, die Organindizes, die Organfunktionsindizes, sowie die Gesamtinzidenz einer Gesundheitsstörung werden dann auf einer Anzeigevorrichtung 7, wie etwa einem LCD-Display zur Information des Anwenders angezeigt (Schritt 104). Gleichzeitig werden dem Anwender zum Vergleich der durchschnittliche Gesundheitsindex, Organindex etc. in der Bevölkerung der gleichen Altersgruppe angezeigt. Darüber hinaus werden dem Benutzer Empfehlungen bzgl. zu ergreifenden Maßnahmen zur Verbesserung seines Gesundheitsindexes angezeigt. Die Daten können aber auch über eine Datenübertragungsvorrichtung 5, wie etwa eine Infrarotschnittstelle, eine Schnittstelle zum Internet oder eine Schnittstelle zum Mobilfunknetz an einen externen Computer übertragen werden. Mittels der Datenübertragungsvorrichtung kann die Abschätzeinrichtung auch ganz allgemein für telemedizinische Anwendungen genutzt werden.

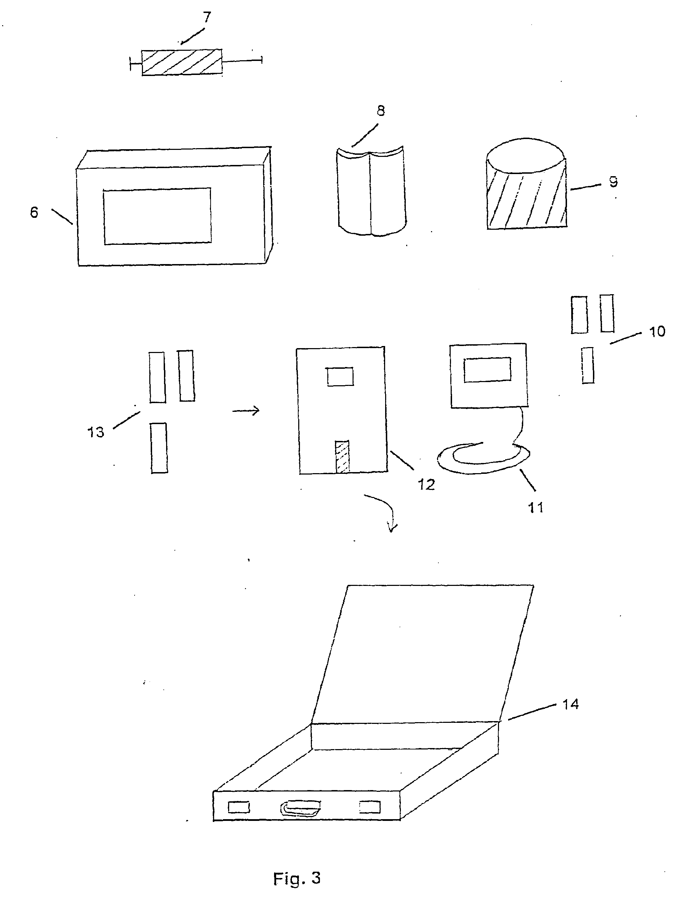

Wie in Fig. 3 gezeigt, kann die Abschätzvorrichtung als Personal Digital Assistant (PDA) 6 ausgeführt sein. Die Abschätzvorrichtung wird dabei zusammen mit zur Bestimmung der Werte der charakteristischen Merkmale notwendigen Meßgeräten und sonstigen Utensilien in einem Köfferchen 14 angeboten. Solche Meßgeräte umfassen ein Glucose-/Cholesterin-Meßgerät 12, ein Handblutdruckgerät 11, Urin-Teststreifen 10, sowie eine nicht gezeigte Körperwaage. Weiterhin enthält das Köfferchen 14 Teststreifen 13 für das Glucose/Cholesterin-Meßgerät 12, Punktionszubehör 9 sowie eine Gebrauchsanweisung 8 zum Betrieb der Abschätzvorrichtung 6 sowie der Messgeräte.

**Patentansprüche**

1. Einrichtung zum Abschätzen einer Wahrscheinlichkeit eines zukünftigen Auftretens bzw. Nichtauftretens einer Gesundheitsstörung bei einem Individuum mit einer Einlesevorrichtung (1) zum Einlesen jeweils einer zugehörigen Ausprägung mindestens eines charakteristischen Merkmals des Individuums, **gekennzeichnet durch** eine Berechnungsvorrichtung (2) zum Berechnen einer Gesamtwahrscheinlichkeit des zukünftigen Auftretens bzw. Nichtauftretens einer von mindestens zwei unterschiedlichen Gesundheitsstörungen aus den eingelesenen Angaben zur jeweiligen Ausprägung des mindestens einen charakteristischen Merkmals.

2. Einrichtung nach Anspruch 1, **dadurch gekennzeichnet, dass** das Auftreten einer Gesundheitsstörung ein zum Tod des Individuums führendes Auftreten der Gesundheitsstörung ist und damit mittels der Berechnungsvorrichtung die Gesamtwahrscheinlichkeit dafür berechenbar ist, dass der Tod des Individuums aufgrund einer von mindestens zwei unterschiedlichen Gesundheitsstörungen zukünftig auftritt bzw. nicht auftritt.

3. Einrichtung nach Anspruch 1 oder 2, **dadurch gekennzeichnet, dass** die Einrichtung weiterhin eine Bereitstellungvorrichtung (2, 3) zum Bereitstellen einer Wahrscheinlichkeitskorrelation zwischen möglichen Ausprägungen des mindestens einen charakteristischen Merkmals und der Wahrscheinlichkeit des zukünftigen Auftretens bzw. Nichtauftretens einer von mindestens zwei vorgegebenen Gesundheitsstörungen aufweist und die Gesamtwahrscheinlichkeit mittels der Berechnungsvorrichtung (2) aus der Wahrscheinlichkeitskorrelation berechenbar ist.

**4.** Einrichtung nach Anspruch 3, **dadurch gekennzeichnet, dass** mittels der Bereitstellungsvorrichtung die Wahrscheinlichkeitskorrelation durch Kombination einer ersten in einer Speichervorrichtung (3) abgespeicherten Einzelwahrscheinlichkeitskorrelation zwischen möglichen Ausprägungen mindestens eines des mindestens einen charakteristischen Merkmals und der Wahrscheinlichkeit des zukünftigen Auftretens bzw. Nichtauftretens mindestens einer ersten vorgegebenen Gesundheitsstörung mit mindestens einer zweiten in der Speichervorrichtung (3) abgespeicherten Einzelwahrscheinlichkeitskorrelation zwischen möglichen Ausprägungen mindestens eines des mindestens einen charakteristischen Merkmals und der Wahrscheinlichkeit des zukünftigen Auftretens bzw. Nichtauftretens einer zweiten vorgegebenen Gesundheitsstörung in der Einrichtung bereitstellbar ist.

**5.** Einrichtung nach Anspruch 3, **dadurch gekennzeichnet, dass** die Bereitstellungsvorrichtung eine Speichervorrichtung (3) zum Abspeichern einer über eine Eingabevorrichtung (1) eingelesenen Wahrscheinlichkeitskorrelation aufweist.

**6.** Einrichtung nach einem der vorausgehenden Ansprüche, **dadurch gekennzeichnet, dass** die mittels der Berechnungsvorrichtung (2) berechenbare Gesamtwahrscheinlichkeit die Wahrscheinlichkeit des zukünftigen Auftretens bzw. Nichtauftretens einer Gesundheitsstörung innerhalb eines vorgegebenen Zeitraums darstellt.

**7.** Einrichtung nach einem der vorausgehenden Ansprüche, **dadurch gekennzeichnet, dass** die Einlesevorrichtung (1) eine Dateneingabevorrichtung (1) zur Eingabe der zugehörigen Ausprägung eines charakteristischen Merkmals des Individuums und/oder eine Meßvorrichtung (10, 11, 12) zum direkten Bestimmen der zugehörigen Ausprägung eines charakteristischen Merkmals am Individuum aufweist.

**8.** Einrichtung nach einem der vorausgehenden Ansprüche, **dadurch gekennzeichnet, dass** mittels der Einlesevorrichtung (1) die zugehörige Ausprägung eines charakteristischen Merkmals des Individuums als Zahlenwert und/oder die zugehörige Ausprägung eines charakteristischen Merkmals des Individuums als qualitative Angabe einlesbar ist.

**9.** Einrichtung nach einem der vorausgehenden Ansprüche, **dadurch gekennzeichnet, dass** mittels der Berechnungsvorrichtung (2) die Gesamtwahrscheinlichkeit des zukünftigen Auftretens bzw. Nichtauftretens einer von nur die Gesundheit eines bestimmten Organs betreffenden Gesundheitsstörungen berechenbar ist und damit die berechnete Gesamtwahrscheinlichkeit die Wahrscheinlichkeit für das Individuum angibt, an dem betreffenden Organ zu erkranken bzw. nicht zu erkranken.

**10.** Einrichtung nach einem der vorausgehenden Ansprüche, **dadurch gekennzeichnet, dass** die mindestens zwei Gesundheitsstörungen mindestens zwei unterschiedliche Organe/Organsysteme betreffen und damit die berechnete Gesamtwahrscheinlichkeit die Wahrscheinlichkeit für das Individuum angibt, an einem der mindestens zwei unterschiedlichen Organe/Organsysteme zu erkranken bzw. nicht zu erkranken.

**11.** Einrichtung nach einem der vorausgehenden Ansprüche, **dadurch gekennzeichnet, dass** mittels der Berechnungsvorrichtung die Gesamtwahrscheinlichkeit des zukünftigen Auftretens bzw. Nichtauftreten einer von bestimmten für die Abschätzung der Gesamtgesundheit des Individuums wesentlichen Gesundheitsstörungen berechenbar ist und damit die berechnete Gesamtwahrscheinlichkeit die Wahrscheinlichkeit für das Individuum angibt, insgesamt gesund bzw. nicht gesund zu bleiben.

**12.** Einrichtung nach Anspruch 11, **dadurch gekennzeichnet, dass** die für die Abschätzung der Gesamtgesundheit des Individuums wesentlichen Gesundheitsstörungen den akuten Herzinfarkt und die Herzschwäche, den Lungen- und Dickdarmkrebs, den Schlaganfall und/oder Diabetes Mellitus, sowie im Falle einer weiblichen Person insbesondere den Brustkrebs der Frau umfassen.

**13.** Einrichtung nach einem der vorausgehenden Ansprüche, **dadurch gekennzeichnet, dass** die Einrichtung weiterhin eine Datenübertragungsvorrichtung (4) zum Empfangen und / oder Versenden von Daten von einem bzw. an einen externen Datenspeicher aufweist.

**14.** Verfahren zum Abschätzen einer Wahrscheinlichkeit eines zukünftigen Auftretens bzw. Nichtauftretens einer Gesundheitsstörung bei einem Individuum, bei dem jeweils eine zugehörige Ausprägung mindestens eines charakteristischen Merkmals des Individuums in eine Abschätzeinrichtung (6) eingelesen wird (102), **dadurch gekennzeichnet, dass** daraus eine Gesamtwahrscheinlichkeit des zukünftigen Auftretens bzw. Nichtauftretens einer von mindestens zwei unterschiedlichen Gesundheitsstörungen berechnet wird (103).

**15.** Computer-Programm mit Programmcode zur Durchführung des Verfahrens nach Anspruch 14, wenn das Programm auf einem Rechner abläuft.

**16.** Computer-Programm-Produkt mit auf einem maschinenlesbaren Träger, insbesondere einer CD, gespeichertem Programmcode zur Durchführung des Verfahrens nach Anspruch 14, wenn das Programm-Produkt auf einem Rechner abläuft.

**Fig. 1**

| | |
|---|---|
| Auswahl der zu berücksichtigenden Gesundheitsstorungen | 101 |
| Eingabe zugehöriger Werte von charakteristischen Merkmalen | 102 |
| Berechnung der Gesamtwahrscheinlichkeit aus Kombination von Einzelwahrscheinlich-keitskorrelationen | 103 |
| Ergebnisanzeige | 104 |

**Fig. 2**

Fig. 3

**Europäisches Patentamt**

# EUROPÄISCHER RECHERCHENBERICHT

Nummer der Anmeldung

EP 05 01 4063

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | US 2003/167188 A1 (HASHIGUCHI TAKESHI ET AL) 4. September 2003 (2003-09-04) * Absätze [0029], [0043], [0050], [0056] * ----- | 1,3-8, 11-16 | A61B5/00 |
| X | US 2003/120515 A1 (GELLER JACOB) 26. Juni 2003 (2003-06-26) * Absätze [0110], [0114], [0115]; Abbildungen 4,5 * ----- | 1,3-12, 14-16 | |
| A | US 4 957 115 A (SELKER ET AL) 18. September 1990 (1990-09-18) * Spalte 1, Zeile 32 - Zeile 36 * * Spalte 3, Zeile 25 - Zeile 30 * ----- | 1,2 | |

|  |
|---|
| RECHERCHIERTE SACHGEBIETE (Int.Cl.7) |
| A61B G06F |

Der vorliegende Recherchenbericht wurde für alle Patentansprüche erstellt

| Recherchenort | Abschlußdatum der Recherche | Prüfer |
|---|---|---|
| Den Haag | 5. Oktober 2005 | Knüpling, M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

.................................................................

& : Mitglied der gleichen Patentfamilie, übereinstimmendes Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 05 01 4063

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-10-2005

| Im Recherchenbericht angeführtes Patentdokument | Datum der Veröffentlichung | Mitglied(er) der Patentfamilie | Datum der Veröffentlichung |
|---|---|---|---|
| US 2003167188 A1 | 04-09-2003 | KEINE | |
| US 2003120515 A1 | 26-06-2003 | KEINE | |
| US 4957115 A | 18-09-1990 | AT 140601 T | 15-08-1996 |
| | | AU 619288 B2 | 23-01-1992 |
| | | AU 3421889 A | 16-10-1989 |
| | | CA 1323431 C | 19-10-1993 |
| | | CN 1038583 A | 10-01-1990 |
| | | DE 68926877 D1 | 29-08-1996 |
| | | DE 68926877 T2 | 28-11-1996 |
| | | EP 0370085 A1 | 30-05-1990 |
| | | JP 2504232 T | 06-12-1990 |
| | | WO 8909022 A1 | 05-10-1989 |

EPO FORM P0461

Für nähere Einzelheiten zu diesem Anhang : siehe Amtsblatt des Europäischen Patentamts, Nr.12/82